# EUROPEAN PATENT APPLICATION

(11) **EP 3 456 839 A1**
(43) Date of publication of application: **20.03.2019**
(21) Application number: 17190993.0
(22) Date of filing: 14.09.2017
(51) Int. Cl.: C12Q 1/68, C12Q 1/04

(54) **METHOD OF ENRICHMENT OF MICRO-ORGANISMS IN A METAGENOMICS WORKFLOW**

(71) Applicant: Siemens Healthcare GmbH, 91052 Erlangen (DE)
(72) Inventor: MITRA, Nivedita, 560066 Bangalore (IN); VUTUKURU, Ramya, 560019 Bangalore, Karnataka (IN)
(74) Representative: Patentanwälte Bals & Vogel

(57) **Abstract**

A method (1) of enriching micro-organisms, in a metagenomics workflow, is disclosed. In an aspect of the invention, the method (1) includes obtaining said sample suspected to contain micro-organisms; concentrating said micro-organisms from said sample; and processing said micro-organisms to obtain genetic information specific to said micro-organisms. In another aspect of the invention, for concentrating said micro-organisms from said sample, the method (1) includes selectively lysing eukaryotic cells present in said sample; removing eukaryotic DNA released from said lysed eukaryotic cells; and isolating said micro-organisms from said sample after removal of said eukaryotic DNA.

## Description

The present invention relates to a method of enrichment of micro-organisms, in a metagenomics workflow, in a sample suspected to contain micro-organisms.

The metagenomics approach of nucleic acid sequencing is an unbiased way to analyze all the nucleic acid present in a sample. In comparison to pre-determined assay based methods of identification of pathogens, the metagenomics approach allows for identification of unsuspected micro-organisms. This enables further analysis of such pathogens which are not detected by traditional genome amplification approaches. The metagenomics approach also allows for the detection of antimicrobial resistance patterns that are distributed throughout the genome length. The presence of an overwhelming amount of human/host genomic deoxyribonucleic acid (DNA) background along with the target microbial DNA is the biggest technical challenge faced in the implementation of next generation sequencing workflows for identification of micro-organisms and their associated antibiotic resistance information from samples such as, but not limited to, blood, sputum, swab, and cerebrospinal fluid.

In a metagenomics study conducted on nasopharyngeal aspirates, disclosed in Yang et. al, J Clinical Microbiology, 49:3463-3469, it was revealed that up to 95% of raw next generation sequencing reads were that of human genomic DNA. In a sample like whole blood, this problem is bigger, due to excessive amount of white blood cells present in a 10 mL blood sample (typical blood size collected to detect 1 cFU/mL pathogens in a sepsis diagnosis). The human genomic DNA present in the sample adds to sequencing time and cost. In addition, the subtraction of human genomic DNA component from that of the target-pathogen DNA is a computationally intensive process. There are multiple enrichment methods and kits available that can selectively eliminate human genomic DNA or selectively enrich pathogens. However, when such methods are used in a metagenomics workflow, adequate removal of human genomic DNA is not assured, thereby resulting in carry-over of significant amount of human genomic DNA. Such carry-over human genomic DNA can be a hindrance when very low-copies of pathogens are to be detected in the sample. If the workflow involves whole genomic amplification methods, there is a high vulnerability of amplification of non-specific DNA. This makes the downstream sequencing and bioinformatics lengthy and cumbersome.

In light of the above, there exists a need to provide a method that removes eukaryotic cells and/or genomic DNA from a sample so as to make a more enriched pathogen sample available for the metagenomics workflow. There also exists a need to provide a method of removal of eukaryotic cells and/or genomic DNA from a sample, which complements the current methods of DNA sequencing.

The object of the invention is therefore to provide a method for enrichment of micro-organisms in a given sample, thereby rendering an enriched pathogen sample for the metagenomics workflow of genomic sequencing.

The invention relates to a method of enrichment of micro-organisms, in a metagenomics workflow, from a sample suspected to contain micro-organisms. The method includes obtaining a sample suspected to contain micro-organisms. Said sample may be obtained, for example, from a patient. The method further comprises concentrating said micro-organisms present in said sample. Concentrating micro-organisms enables removal of eukaryotic DNA background and efficient analysis of said micro-organisms. In a further step, the method comprises processing said micro-organisms to identify genetic information pertaining to said micro-organisms. Obtaining genetic information of said micro-organisms enables determining, for example, the antibiotic resistance profile of said micro-organisms.

In a preferred embodiment of the invention, in concentrating said micro-organisms from said sample, the method further comprises selectively lysing eukaryotic cells present in said sample. Selective lysis is a method of breaking down cells of a particular type and/or species without damaging the cells of another type and/or species. Therefore, the eukaryotic cells are selectively lysed such that said micro-organisms that may be present in said sample are not affected. On lysis of said eukaryotic cells, eukaryotic nucleic acids are released into said sample. Selectively lysing eukaryotic cells is a negative enrichment method. A negative enrichment method involves capturing and/or non-target cells such that target cells in the sample are intact and unaffected. Further the method includes a step of removing said eukaryotic nucleic acids from said sample. The removal of said eukaryotic nucleic acid is essential so as to enable enrichment of micro-organisms present in said sample. The concentration of micro-organisms in said given sample may be very low. Therefore, any hindrance from eukaryotic nucleic acids is removed. The method further includes a step of isolating said micro-organisms from said sample. Said micro-organisms are separated from said sample for further enrichment. Isolation of micro-organisms from said sample enables elimination of eukaryotic DNA background, thereby enhancing downstream processing of said micro-organisms.

According to an embodiment of the invention, said eukaryotic cells may be lysed using a selective lysis buffer. According to a preferred embodiment of the invention, said selective lysis buffer is a non-ionic detergent. Detergents create pores in the cell-membrane of the cells by solubilizing lipids and proteins in the membrane. Non-ionic detergents are characterized by their uncharged, hydrophilic head-groups. Non-ionic detergents are typically based on polyoxyethylene or a glycoside. Some of the non-ionic detergents that may be used for selective lysis include but are not limited to Triton-X, Tween, and the Brij series. The pH of said non-ionic detergent may be around 8.0 and above. The selective lysis buffer may be used to lyse the eukaryotic cells along with a chaotropic agent. A chaotropic agent is a molecule that can disrupt the hydrogen bonding in macromolecules. Chaotropic agents affect the stability of macromolecules by weakening the hydrophobic effect. A chaotropic agent that may be used along with the selective lysis buffer may be, for example but not limited to, guanidine thiocyanate. The use of a chaotropic agent along with a selective lysis buffer allows for efficient lysis of the cell membrane of said eukaryotic cells, thereby releasing eukaryotic DNA into said sample.

According to an embodiment of the invention, said eukaryotic DNA which is released from said lysed eukaryotic cells may be removed using one or more enzymes that lyse DNA. Removal of said eukaryotic DNA forms a part of negative enrichment of micro-organisms. Such an enzyme may be, for example, nuclease and more particularly, deoxyribonuclease (DNAse). DNAse is an enzyme that catalyzes the hydrolytic cleavage of phosphodiester linkages in the DNA backbone, thereby breaking down DNA. The use of enzymes that break down nucleic acids eliminates eukaryotic DNA background and provides for efficient characterization of said micro-organisms that may be present in said sample.

According to a further embodiment of the invention, said broken down eukaryotic DNA may be removed by centrifuging the sample. Said sample may be centrifuged at a relative centrifugal force ranging between 5000 x g and 7000 x g. When said sample is centrifuged, said eukaryotic DNA settles down in the pellet formed at the bottom of the centrifuge tube. The supernatant containing said micro-organisms is extracted and may be subjected to further enrichment.

According to another embodiment of the invention, said eukaryotic DNA released from said selectively lysed eukaryotic cells may be denatured in alkaline conditions. DNA may be denatured using alkaline agents such as, but not limited to, sodium hydroxide (NaOH). Said alkaline agent may have a pH of 8.0 or above. Alkaline agents denature DNA by changing the pH and removing protons that contribute to hydrogen-bonds, thereby enabling removal of eukaryotic DNA background from said sample.

According to a further embodiment of the invention, said denatured eukaryotic DNA may be removed from said sample by filtering said denatured sample. Said denatured sample may be filtered using filters made of, for example, glass fiber or polycarbonate, with a pore size in the range between 0.1 µm to 0.5 pm. Said intact micro-organisms are captured on said filter, whereas said eukaryotic genomic DNA passes through said filter. Said micro-organisms captured on said filter may be lysed and used for further downstream processing.

According to yet another embodiment of the invention, said lysed eukaryotic cells may be removed from said sample by using one or more DNA binding proteins which bind specifically to eukaryotic DNA. Said DNA binding proteins may be, for example, histone proteins or proteins that bind to methylated DNA. Said DNA binding proteins may be coated on to a matrix such as paramagnetic beads such that once eukaryotic DNA is bound to said proteins, such formed complex is removed using a magnet. The use of DNA binding proteins enables removal of any eukaryotic DNA that may be remaining in said sample.

According to a preferred embodiment of the invention, said micro-organisms are isolated from said enriched sample by incubating said sample with at least one matrix coated with a substrate having affinity for micro-organisms. Isolation of micro-organisms from said sample is a positive enrichment method. A positive enrichment method involves isolation of target cells from a given sample, leaving behind the non-target cells and cellular components in the sample. Said substrate may be, for example, Apolipoprotein H. When a matrix coated with said substrate is brought in contact with said sample, the micro-organisms present in said sample bind to said substrate to form a complex. Apolipoprotein H allows for concentration of micro-organisms from said sample.

According to an embodiment of the invention, said matrix on to which said substrate is coated is chosen from a group which includes but is not limited to paramagnetic beads, polystyrene beads, and glass beads.

According to an embodiment of the invention, if said matrix is a paramagnetic bead, said complex formed is isolated from said sample using a magnet. Said paramagnetic bead is attracted to said magnet, thereby enabling easy isolation of said complex.

According to an embodiment of the invention, if said matrix is a polystyrene bead or glass bead, said complex formed is isolated from said sample using centrifugation. On centrifugation, said complex settles down in the pellet. Therefore, said complex may be extracted to obtain said complex.

According to an embodiment of the invention, said sample is chosen from a group comprising but not limited to whole blood, sputum, urine, cerebrospinal fluid and bronchoalveolar lavage.

The present invention also provides for a kit for carrying out the abovementioned method. According to the invention, said kit includes a selective lysis buffer, one or more enzymes for lysis of eukaryotic DNA, an alkaline agent, at least one filter, and one or more matrices coated with a substrate having affinity for micro-organisms, which can be used for the enrichment of micro organisms present in a sample.

The present invention is further described hereinafter with reference to illustrated embodiments shown in the accompanying drawings, in which:
- FIG 1: illustrates a schematic diagram of a flow chart of an embodiment of a method according to the invention.
- FIG 2a: illustrates a graph depicting a comparison of change in the amounts of eukaryotic DNA with employment of enrichment steps according to the invention.
- FIG 2b: illustrates a graph depicting a comparison of change in the amounts of micro-organism DNA between a saline sample with no human gDNA background (unenriched) and enriched blood sample having micro-organism concentration of 100 CFU/milliliter.
- FIG 2c: illustrates a graph depicting a comparison of change in the amounts of micro-organism DNA between a saline sample with no human gDNA background (unenriched) and enriched blood sample having micro-organism concentration of 10 CFU/milliliter.
- FIG 3: illustrates a graph depicting the change in the amounts of eukaryotic DNA to microbial DNA with the employment of enrichment workflow steps according to the invention.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

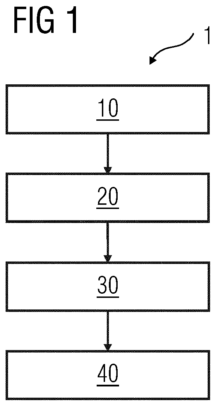

FIG 1 illustrates a flowchart of an embodiment of a method 1 of enrichment of micro-organisms, in a metagenomics workflow, according to the invention. In an embodiment, a whole blood sample suspected to contain micro-organisms is used for enrichment. The sample volume may be in the range between 8 milliliter and 15 milliliter. In the present embodiment, a sample volume of 8 milliliter is considered for enrichment. Said whole blood sample contains eukaryotic cells such as red blood cells and white blood cells among other components. At step 10, said eukaryotic cells present in said whole blood sample are selectively lysed. The step of selective lysis is performed such that said micro-organisms present in said sample are not affected. Said eukaryotic cells are lysed using a selective lysis buffer. In an embodiment, said selective lysis buffer is a non-ionic detergent such as Triton X having a pH around 8.0 or above. Non-ionic detergents affect the cell membrane of eukaryotic cells by creating pores and thereby disintegrating the cell membrane. In an alternate embodiment, the eukaryotic cells are selectively lysed by using a chaotropic agent, for example, guanidine thiocyanate, in combination with a selective lysis buffer. Guanidine thiocyanate breaks down the hydrogen bonds thereby affecting the stability of the native state of macromolecules. On selective lysis of eukaryotic cells, eukaryotic DNA is released into said sample.

Said eukaryotic DNA released into said sample is removed at step 20 of said method 1, so as to eliminate the eukaryotic DNA background. In an embodiment, one or more enzymes, such as nucleases, that breakdown eukaryotic DNA are used to enable removal of eukaryotic DNA. Nucleases break the bonds between nucleotides, particularly the phosphodiester bonds, in nucleic acids. Nucleotides are small subunits that form DNA or RNA. Nucleases are non specific and cut the DNA strand at any random location in an unbiased manner. DNAses that work in the presence of guanidine thiocyanate are used for breaking down eukaryotic DNA released on lysis of said eukaryotic cells. Said broken down DNA is then removed from said sample by centrifuging said sample at a relative centrifugal force ranging between 5000 x g and 7000 x g. By centrifuging said sample, said micro-organisms settle down in the pellet at the bottom of the centrifuge tube. Therefore, said eukaryotic DNA settles down in a pellet that is formed as a result of centrifugation. Said supernatant is removed from said sample so as to obtain an enriched sample containing micro-organisms. In an alternative embodiment, removal of said eukaryotic DNA is achieved by using an alkaline selective lysis buffer so as to denature said eukaryotic DNA. Said denatured eukaryotic DNA is removed from the said sample, by filtering the selectively lysed sample to capture the intact pathogens on a filter for further downstream analysis.

Said supernatant containing micro-organisms is brought in contact with one or matrix coated with at least one DNA binding protein so as to remove any micro organisms that may be left out in said sample. Said DNA binding protein used in the present embodiment is histone proteins. Histone proteins have basic amino acids which bind to the acidic phosphate groups of DNA. Alternatively, said DNA binding protein may be any protein that binds to methylated DNA such as methyl-CpG-binding domains (MBD Proteins) or immune proteins known to bind micro-organisms. Said histone proteins are coated onto a matrix. Said matrix preferably is spherical is shape, thereby providing higher surface area for binding of said eukaryotic DNA. A paramagnetic bead is chosen as a matrix for capturing said eukaryotic DNA that may be present in said sample. Alternatively, said matrix is chosen from a group comprising, but not limited to, magnetic, ferromagnetic, ferromagnetic, polystyrene or glass bead. Said histone proteins when brought in contact with said eukaryotic DNA, bind to said eukaryotic DNA to form a complex. As said matrix used is a paramagnetic bead, said complex is removed from the sample using a magnet.

Once said eukaryotic DNA is removed from said sample, said micro-organisms present in said pellet are re-suspended into a buffer. At step 30, said micro-organisms are incubated with at least one matrix coated with one or more proteins that specifically bind to micro-organisms. In the present embodiment, said protein is Apolipoprotein H. Apolipoprotein H is also known as beta2-glycoprotein I and is a plasma glycoprotein. Apolipoprotein H has high affinity for micro-organisms or their proteins. Alternatively, said one or more proteins coated on the beads can be any immune protein that binds non-specifically to any micro-organism. Said matrix is preferably spherical in shape and is a paramagnetic bead. Alternatively, said matrix is chosen from a group comprising, but not limited to, magnetic, ferromagnetic, ferromagnetic, polystyrene or glass bead. Said paramagnetic bead coated with Apolipoprotein H is brought in contact with said micro-organisms and incubated for a time period ranging between 5 minutes and 30 minutes, and more particularly between 5 minutes and 15 minutes. Said micro-organisms present in said sample bind to said matrix and form a complex. Said complex is removed from the sample using a magnet. Said micro-organisms bound to Apolipoprotein H can be lysed so as to extract micro-organism DNA for further downstream processing at step 40. Such further processing may involve, for example, using quantitative polymerase chain reaction. One of the significant steps in downstream processing of said micro-organism DNA could be identifying the sequence of said micro-organism DNA. Such sequencing may be performed using nanopore based sequencer, for example. In one instance, genomic sequencing enables identification of the antibiotic resistance profile of said micro-organisms.

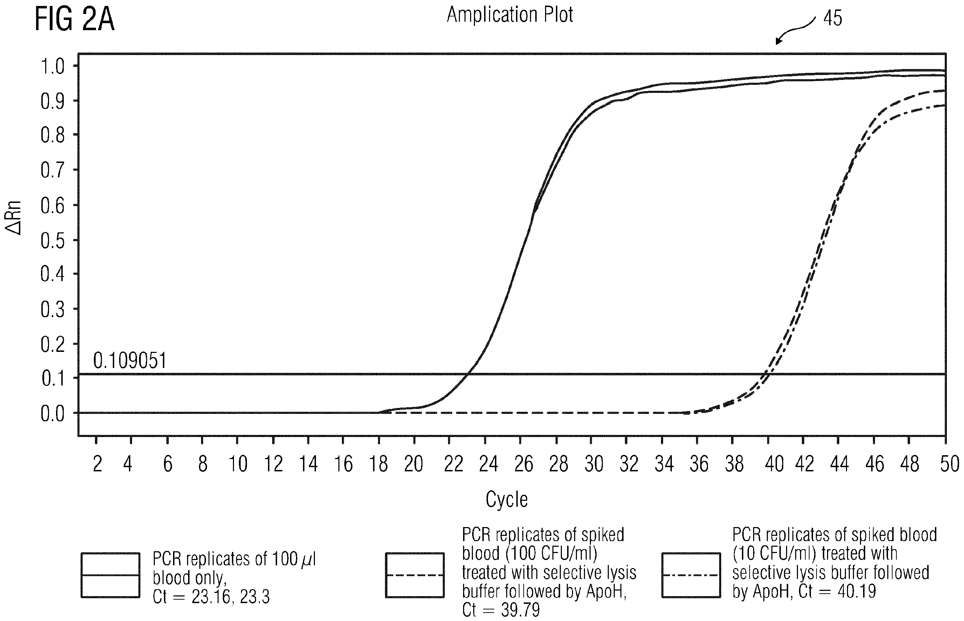

FIG 2A illustrates a graph 45 depicting a change in the amount of eukaryotic DNA with employment of enrichment steps according to the invention. Said enriched sample was checked for the presence of eukaryotic DNA by performing a quantitative polymerase chain reaction. The primer used for the amplification of eukaryotic DNA is progestagen-associated endo-metrial protein (PAEP) human DNA marker. The samples used in the quantitative polymerase chain reaction include a 100 micro liter of non-enriched whole blood sample, wherein said 100 micro liter of non-enriched whole blood sample represents said starting 8 milliliter blood volume; 100 micro liter of enriched whole blood sample (8 milliliter blood sample is concentrated to 100 micro liter after enrichment workflow) containing *Candida tropicalis* at a concentration of 100 colony forming units (CFU)/milliliter; and 10 micro liter of enriched whole blood sample containing *Candida tropicalis* at a concentration of 100 CFU/milliliter. The X-axis of the graph 45 represents the number of cycles of polymerase chain reaction for amplification of DNA present in the sample. The Y-axis of the graph 45 represents the amount of fluorescence produced and detected as a result of amplification of nucleic acids. The amount of fluorescence generated is directly proportional to the amount of DNA present in the sample being amplified. Therefore, as the polymerase chain reaction progresses, the amount of nucleic acids in the sample increases. If the amount of nucleic acid present in the sample is low, more number of cycles of polymerase chain reaction would be required to produce sufficient amount of amplified DNA. Therefore, greater number of amplification cycles would be required for generation of fluorescence beyond a certain threshold. In the present embodiment, the threshold of fluorescence is 0.109051.

As depicted in said graph 45, when said non-enriched whole blood sample is amplified, the number of amplification cycles required to produce fluorescence above the threshold value is 23.16 and 23.3. Therefore, when the quantity of non-enriched whole blood sample is scaled up to 8000 micro liter, the number of amplification cycles required to produce fluorescence above threshold level is 16.98. When a 100 micro liter enriched whole blood sample containing *Candida tropicalis* at a concentration of 100 CFU/milliliter is amplified, the number of amplification cycles required to produce fluorescence above the threshold level is 39.79. The delta in the threshold cycle between amplification of non-enriched whole blood sample and enriched whole blood sample is 22.81. Therefore, an enrichment fold (ratio of eukaryotic DNA before enrichment and eukaryotic DNA after enrichment) of 7.36X10⁶ is achieved.

The graph 45 further depicts that when an enriched whole blood sample containing *Candida tropicalis* at a concentration of 10 CFU/milliliter is amplified, the number of amplification cycles required to produce fluorescence above the threshold level is 40.19. The delta in the threshold cycle between amplification of non-enriched whole blood sample (CT value has been estimated for 8ml non-enriched whole blood sample from the CT value of the 100 micro liter fraction) and enriched whole blood sample is 23.21. Therefore, an enrichment fold (ratio of eukaryotic DNA before enrichment and eukaryotic DNA after enrichment) of 9.72X10⁶ is achieved. Hence, irrespective of the spiked in concentrations of *Candida tropicalis,* the depletion fold achieved for eukaryotic DNA is around the same.

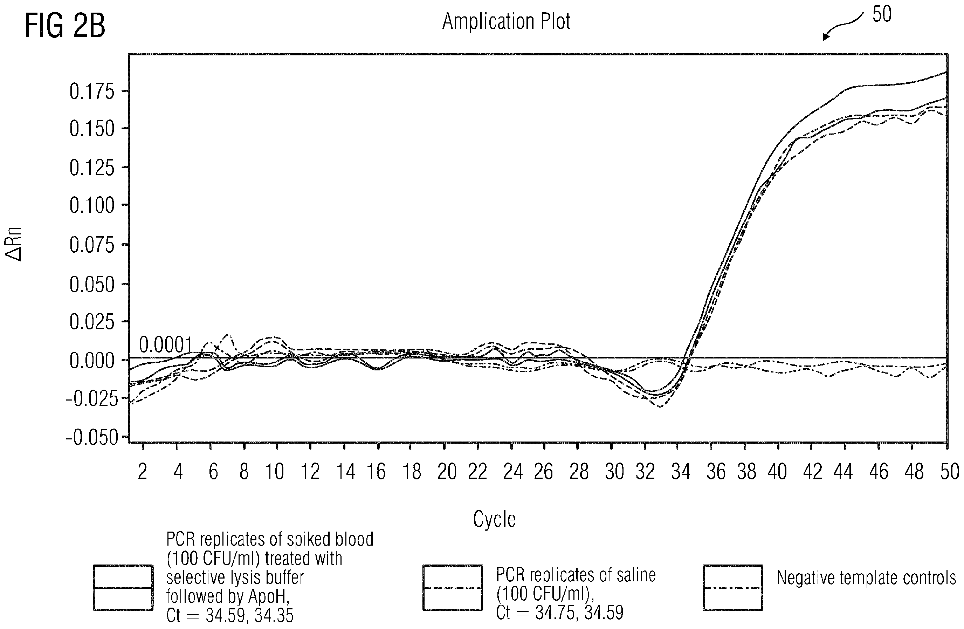

FIG 2B illustrates a graph 50 depicting a change in the amounts of micro-organism DNA between a saline sample and enriched sample having micro-organism concentration of 100 CFU/milliliter. The enriched sample was checked for the presence of micro-organism DNA by performing a quantitative polymerase chain reaction. The micro organism in the present embodiment is *Candida tropicalis.* The primer used for the amplification of micro-organism DNA is *Candida tropicalis* 18S rRNA marker. The samples used in the quantitative polymerase chain reaction include a 100 micro liter of saline sample containing *Candida tropicalis* at a concentration of 100 CFU/milliliter, 100 micro liter of enriched whole blood sample containing *Candida tropicalis* at a concentration of 100 colony forming units (CFU)/milliliter and negative template controls. The X-axis of the graph 50 represents the number of cycles of polymerase chain reaction for amplification of DNA present in the sample. The Y-axis of the graph 50 represents the amount of fluorescence produced and detected as a result of amplification of nucleic acids. As depicted in said graph 50, when a 100 micro liter of said saline sample is amplified, the number of amplification cycles required to produce fluorescence above the threshold value is 34.75 and 34.59. When a 100 micro liter enriched whole blood sample containing *Candida tropicalis* at a concentration of 100 CFU/milliliter is amplified, the number of amplification cycles required to produce fluorescence above the threshold level is 34.59 and 34.35. Therefore, the enrichment steps do not affect the quantity of micro-organism cells and therefore micro-organism DNA. Thus, the loss of micro-organisms as a result of the enrichment steps is negligible.

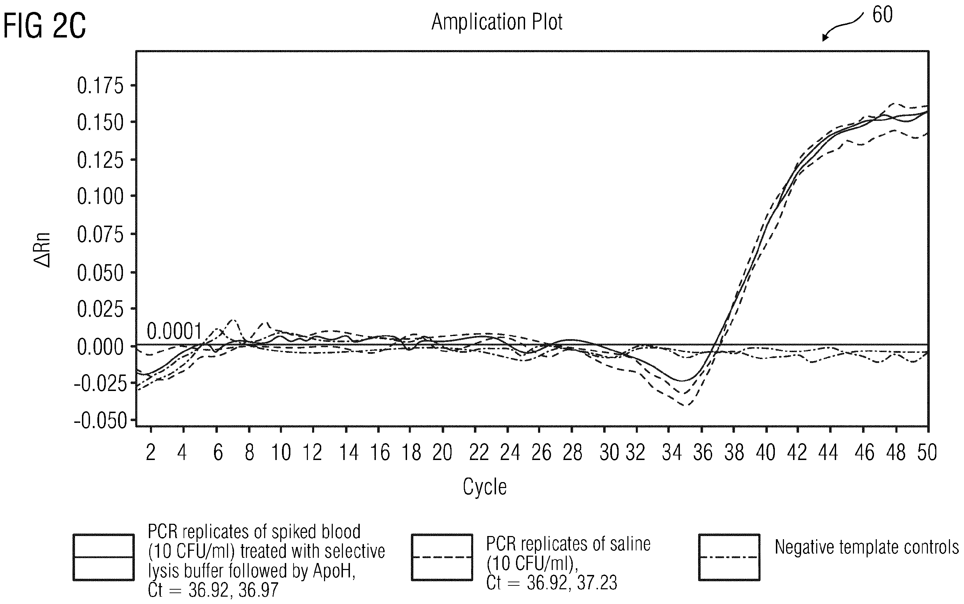

FIG 2C illustrates a graph 60 depicting a comparison of change in the amounts of micro-organism DNA between a saline sample and enriched sample having micro-organism concentration of 10 CFU/milliliter. The enriched sample was checked for the presence of micro-organism DNA by performing a quantitative polymerase chain reaction. The primer used for the amplification of micro-organism DNA is *Candida tropicalis* 18S rRNA marker. The samples used in the quantitative polymerase chain reaction include a 100 micro liter of saline sample containing *Candida tropicalis* at a concentration of 10 CFU/milliliter, 100 micro liter of enriched whole blood sample containing *Candida tropicalis* at a concentration of 100 colony forming units (CFU)/milliliter and negative template controls. As depicted in said graph 60, when a 100 micro liter of said saline sample is amplified, the number of amplification cycles required to produce fluorescence above the threshold value is 36.92 and 37.23. When a 100 micro liter enriched whole blood sample containing *Candida tropicalis* at a concentration of 10 CFU/milliliter is amplified, the number of amplification cycles required to produce fluorescence above the threshold level is 36.92 and 36.97.

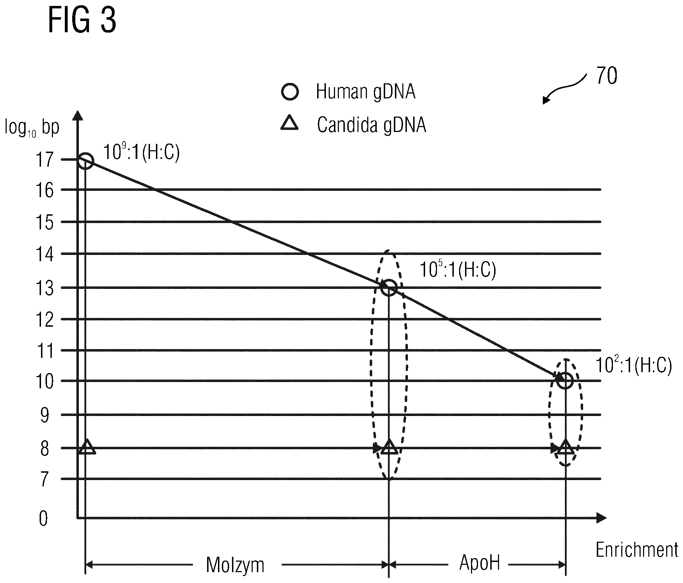

FIG 3 illustrates a graph 70 depicting the change in the amounts of eukaryotic DNA to microbial DNA with the employment of enrichment steps according to the invention. The X-axis of graph 70 depicts the progression of the enrichment steps and the Y-axis depicts length of DNA (in base pairs) of DNA present in the sample. According to said graph 70, as the enrichment steps progress, the amount of eukaryotic DNA or human genomic DNA is reduced. However, the concentration of micro-organism DNA or *Candida* DNA is not affected by the enrichment steps. The ratio of human genomic DNA to *Candida* DNA, after the treatment of said sample with selective lysis buffer, reduces from 10⁹:1 to 10⁵:1. Further, after said sample is treated with Apolipoprotein H, the ratio of human genomic DNA to *Candida* DNA further reduces from 10⁵:1 to 10²:1.

The enriched *Candida* DNA is further used for genomic amplification and DNA sequencing, at step 40.

The instant teachings also provide kits designed to expedite performance of the subject methods. Kits serve to expedite the performance of the methods of interest by assembling two or more components required for carrying out the disclosed methods. Kits may contain components in pre-measured unit amounts to minimize the need for measurements by end-users. Kits may include instructions for performing one or more of the disclosed methods. Preferably, said kit components are optimized to operate in conjunction with one another.

In an embodiment, kits comprise a selective lysis buffer for lysing one or more eukaryotic cells in said sample. Said kit further includes one or more enzymes to lyse the released eukaryotic DNA. In an alternate embodiment, said kit comprises an alkaline reagent to denature eukaryotic DNA. Said kit further comprises a filter for removal of broken down or denatured eukaryotic DNA. Said kit also includes one or matrices coated with a substrate that has an affinity for micro-organisms. In an alternate embodiment, said kit may further comprise a chaotropic agent. Said chaotropic agent, when use along with said selective lysis buffer, enables efficient lysis of eukaryotic cells.

The abovementioned method of enrichment of micro-organisms involves a combination of negative and positive enrichment methods. Negative enrichment ensures elimination of non-target cells from the sample. Therefore, the non-target background is removed thereby providing a concentrated sample containing target cells. When followed by a positive enrichment step, the target cells are separated from any non-target cells that may have been left out in the process of negative enrichment. Therefore, the combination of negative and positive enrichment steps yields a highly enriched and concentrated sample of target cells, which are micro-organisms, in the present embodiment. The abovementioned method of enrichment of micro-organisms can be integrated efficiently with the downstream sample preparation methods. The method enables nearly complete removal of eukaryotic DNA background, thereby making microbial genomic amplification more efficient. The method also reduces the time taken for DNA sequencing as most of sequences being analyzed are of microbial origin. Thus, this enrichment method can be integrated with the sample preparation methods of any gene sequencing workflow that is used for metagenomics analysis of micro-organisms in a complex sample such as whole blood, sputum, urine, and cerebrospinal fluid.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein; rather, the invention extends to all functionally equivalent structures, methods and uses, such as are within the scope of the appended claims. Those skilled in the art, having the benefit of the teachings of this specification, may effect numerous modifications thereto and changes may be made without departing from the scope and spirit of the invention in its aspects.

### List of reference numbers

1 Method of enrichment of micro-organisms in a metagenomics workflow
45 Graph depicting a change in the amount of eukaryotic DNA with employment of enrichments steps
50 Graph depicting change in the amount of micro-organism DNA between saline sample and enriched sample

## Claims

1. A method (1) of enrichment of micro-organisms, in a metagenomics nucleic acid sequencing workflow, from a sample suspected to contain micro-organisms, said method comprising:
a. obtaining said sample suspected to contain micro-organisms;
b. concentrating said micro-organisms from said sample; and
c. processing said concentrated micro-organisms to obtain genetic information specific to said micro-organisms;
**characterized in that** the concentrating said micro-organisms from said sample further comprises:
d. selectively lysing eukaryotic cells present in said sample;
e. removing eukaryotic DNA released from said lysed eukaryotic cells; and
f. isolating said micro-organisms from said sample after removal of said eukaryotic DNA.

2. The method (1) according to claim 1, wherein the method step d of selectively lysing of the eukaryotic cells comprises the usage of a selective lysis buffer.

3. The method (1) according to claim 2, wherein said selective lysis buffer has a pH of 8.0 or above, preferably a pH of 9, 9.5, and 10.

4. The method (1) according to claim 1, wherein the method step e of removal of said eukaryotic DNA released from said lysed eukaryotic cells comprises the usage of one or more enzymes that lyse DNA.

5. The method (1) according to claim 4, wherein said lysed eukaryotic DNA is removed by centrifuging said sample.

6. The method (1) according to one of the claims 1-3, wherein the method step e of removal of said eukaryotic DNA released from said lysed eukaryotic cells comprises denaturation of said eukaryotic DNA using one or more alkaline agents.

7. The method (1) according to claim 6, wherein said denatured DNA is removed using a filter.

8. The method (1) according to claim 1, wherein the method step f of isolating said micro-organisms from said sample comprises incubation of said sample with at least one matrix coated with a substrate having affinity for micro-organisms to form a complex.

9. The method (1) according to claim 8, wherein said matrix is chosen from a group comprising magnetic beads, ferromagnetic beads, paramagnetic beads, polystyrene beads, and glass beads.

10. The method (1) according to claim 9, wherein said formed complex is isolated using a magnet if said matrix is a magnetic, ferromagnetic or paramagnetic bead.

11. The method (1) according to claim 8 and 9, wherein said formed complex is isolated using centrifugation if said matrix is a polystyrene or glass bead.

12. The method (1) according to claim 1, wherein said sample is chosen from a group comprising but not limited to whole blood, sputum, urine, cerebrospinal fluid, and bronchoalveolar lavage.

13. A kit for carrying out a method (1) of enrichment of micro-organisms in a metagenomics workflow, said kit comprising:
a. a selective lysis buffer for lysis of eukaryotic cells; and
b. one or more matrices coated with a substrate having affinity for micro-organisms for isolation of micro-organisms.

14. The kit according to claim 13, further comprising one or more enzymes for lysis of eukaryotic DNA released from said lysed eukaryotic cells.

15. The kit according to claim 13, further comprising:
a. an alkaline agent for denaturing eukaryotic DNA released from said eukaryotic cells; and
b. at least one filter for removing said denatured eukaryotic DNA.

16. The use of said kit as claimed in claims 13-15 to carry out a method (1) of enrichment of micro-organisms in a metagenomics workflow as claimed in claim 1 to 12.
